# EUROPEAN PATENT SPECIFICATION

(11) **EP 3 626 278 B1**
(45) Date of publication and mention of the grant of the patent: **15.11.2023**
(21) Application number: 18817224.1
(22) Date of filing: 14.06.2018
(51) Int. Cl.: A61M 1/14, G16H 10/00, G16H 20/40, G16H 40/63, G16H 50/20, G16H 50/70, A61M 1/16

(54) **BLOOD PURIFICATION SYSTEM**
BLUTREINIGUNGSSYSTEM
SYSTÈME DE PURIFICATION DE SANG

(30) Priority: 14.06.2017 JP 2017116956
(43) Date of publication of application: 25.03.2020
(73) Proprietor: Nikkiso Company Limited, Tokyo 150-6022 (JP)
(72) Inventor: AOTA Naoyuki, Makinohara-shi Shizuoka 421-0496 (JP); TOYODA Masahiro, Makinohara-shi Shizuoka 421-0496 (JP); HAGIWARA Yasunobu, Makinohara-shi Shizuoka 421-0496 (JP)
(74) Representative: von Hirschhausen, Helge
(86) International application number: PCT/JP2018/022705
(87) International publication number: WO 2018/230635

(56) References cited:
- WO-A1-2016/113069
- JP-A- 2008 176 623
- JP-A- 2014 004 194
- JP-A- 2015 519 133
- US-A1- 2015 148 697
- US-A1- 2016 175 508

## Description

### Technical Field

The present invention relates to a blood purification system including a blood purification apparatus capable of giving blood purification treatment to a patient, and a managing apparatus capable of communicating with the blood purification apparatus in such a manner as to transmit and receive information on the blood purification treatment to and from the blood purification apparatus.

### Background Art

A dialysis apparatus as a blood purification apparatus is used in dialysis treatment or the like. A dialysis room in a medical facility such as a hospital is provided with a plurality of dialysis apparatuses so that dialysis treatment (blood purification treatment) can be given to many patients in the dialysis room. As disclosed by Japanese Unexamined Patent Application Publication No. 2014-4194 for example, each of such dialysis apparatuses is connected to a central monitoring apparatus (a managing apparatus) including a server, and is capable of receiving various pieces of information on patients that are stored in the server. In recent cases, pieces of information on dialysis treatment (for example, patients' past treatment data and so forth) are stored in the server of the central monitoring apparatus, and any of those pieces of information is displayed on a display unit according to need so that a medical worker such as a doctor can grasp the information.

US 2016/175508 A1 relates to real-time spectrophotometric monitoring of spent dialysate by taking into account the effect of varying treatment parameters like the blood flow rate on the detected concentration, so that the progress of one treatment session can be monitored accurately even when treatment conditions change during one particular treatment session. This is accomplished by measuring and storing the concentration of the spent dialysate under each treatment condition in advance, i.e. at the beginning of the treatment session.

US 2015/148697 A1 relates to and aims to predict the risk of live-threatening arrhythmia occurring during dialysis earlier by displaying physiological parameters of the patient that are detected by an implantable medical device during dialysis.

### Summary of Invention

### Technical Problem

Such a known blood purification system can display information stored in the server and can make the medical worker or the like grasp the information. However, depending on the purpose of extraction, pieces of information stored as histories from which any desired pieces of information are to be extracted may include histories that are not suitable as the object of search. In such a case, the pieces of information extracted and displayed may be inappropriate in terms of the purpose of extraction. Consequently, the reliability of information may be deteriorated. In contrast, if such histories are deleted from the storage, actually necessary pieces of information cannot be extracted when searching is performed for another purpose of extraction.

The present invention has been conceived in view of the above circumstances and provides a blood purification system that is capable of effectively utilizing histories stored during blood purification treatment and that helps realize history search suitable for individual purposes of extraction, thereby increasing the reliability of information to be extracted and displayed.

### Solution to Problem

According to the invention of Claim 1, there is provided a blood purification system including a blood purification apparatus that gives blood purification treatment to a patient; a managing apparatus that communicates with the blood purification apparatus in such a manner as to transmit and receive information on the blood purification treatment to and from the blood purification apparatus, the managing apparatus including a storage unit that stores the information on the blood purification treatment as a history for each treatment session in a time course with progress of the treatment; and a display unit provided to the blood purification apparatus or to the managing apparatus and that displays the information on the blood purification treatment. The blood purification system includes an extracting unit that searches for and extracts any history satisfying a predetermined condition from among the histories stored in the storage unit, an excluding unit that excludes any of the histories stored in the storage unit from an object of search performed by the extracting unit, and a display control unit that controls various pieces of information based on the history extracted by the extracting unit to be displayed on the display unit.

According to the invention of Claim 2, in the blood purification system according to Claim 1, the excluding unit is configured such that any item to be excluded from the object of search is specified.

According to the invention of Claim 3, in the blood purification system according to Claim 2, the excluding unit is configured such that the item to be excluded from the object of search is specified in units of one history.

According to the invention of Claim 4, in the blood purification system according to Claim 2, the excluding unit is configured such that the item to be excluded from the object of search is specified in units of one piece of data included in each of the histories.

Further according to the invention of Claim 1, the histories each include a particular incident related to the patient's behavior or the patient's condition having occurred unsteadily in the blood purification treatment. Furthermore, the extracting unit is capable of extracting the particular incident by searching the histories stored in the storage unit except the history excluded by the excluding unit. Furthermore, the blood purification system includes a calculating unit that calculates a time when the particular incident extracted by the extracting unit occurs a predetermined or more number of times as a frequent-occurrence time slot. Furthermore, the display control unit controls the frequent-occurrence time slot calculated by the calculating unit to be displayed on the display unit during a current session of blood purification treatment.

According to the invention of Claim 1, the blood purification system includes the extracting unit that searches for and extracts any history satisfying a predetermined condition from among the histories stored in the storage unit, the excluding unit that excludes any of the histories stored in the storage unit from the object of search performed by the extracting unit, and the display control unit that controls various pieces of information based on the history extracted by the extracting unit to be displayed on the display unit. Therefore, the histories stored during the blood purification treatment can be utilized effectively. Furthermore, history search suitable for individual purposes of extraction is realized, whereby the reliability of information to be extracted and displayed can be increased.

According to the invention of Claim 2, the excluding unit is configured such that any item to be excluded from the object of search is specified. Therefore, the medical worker can precisely determine whether the exclusion of the item by the excluding unit matches the purpose of extraction. Consequently, the reliability of information to be extracted and displayed can be increased further.

According to the invention of Claim 3, the excluding unit is configured such that the item to be excluded from the object of search is specified in units of one history. Therefore, the entirety of the history of a single treatment session can be excluded from the object of search. Hence, information that is unfavorable for performing highly accurate extraction can be excluded in units of one treatment session.

According to the invention of Claim 4, the excluding unit is configured such that the item to be excluded from the object of search is specified in units of one piece of data included in each of the histories. Therefore, the object of exclusion can be specified more precisely.

Further according to the invention of Claim 1, the histories each include the particular incident related to the patient's behavior or the patient's condition having occurred unsteadily in the blood purification treatment. Furthermore, the extracting unit is capable of extracting the particular incident by searching the histories stored in the storage unit except the history excluded by the excluding unit. Furthermore, the blood purification system includes the calculating unit that calculates the time when the particular incident extracted by the extracting unit occurs a predetermined or more number of times as the frequent-occurrence time slot. Furthermore, the display control unit controls the frequent-occurrence time slot calculated by the calculating unit to be displayed on the display unit during the current session of blood purification treatment. Therefore, history search suitable for individual purposes of extraction is realized, whereby the reliability of information to be extracted and displayed can be increased. Furthermore, the medical worker can grasp the frequent-occurrence time slot of the particular incident in advance.

### Brief Description of Drawings

[Fig. 1] Fig. 1 is a block diagram of a blood purification system according to a first embodiment of the present invention.
Fig. 2 is a diagram illustrating an appearance of a blood purification apparatus included in the blood purification system.
Fig. 3 is a schematic diagram illustrating an internal configuration and a blood circuit of the blood purification apparatus included in the blood purification system.
Fig. 4 is a table for describing extraction performed by an extracting unit and calculation performed by a calculating unit in the blood purification system.
Fig. 5 is a table for describing extraction performed by the extracting unit and calculation performed by the calculating unit in the blood purification system.
Fig. 6 is a table for describing extraction performed by the extracting unit and calculation performed by the calculating unit in the blood purification system.
Fig. 7 is a graph illustrating items controlled to be displayed by a display control unit included in the blood purification system.
Fig. 8 is a diagram illustrating other items controlled to be displayed by the display control unit included in the blood purification system.
Fig. 9 is a block diagram of a blood purification system according to a second embodiment of the present invention.
Fig. 10 is a schematic diagram illustrating how a transition of a particular parameter that is estimated by the estimating unit, included in the blood purification system, to be observed after the current time in the current session of blood purification treatment is displayed.
Fig. 11 is a schematic diagram illustrating how a transition of a particular parameter that is estimated by the estimating unit, included in the blood purification system, to be observed after a past time before the current time in the current session of blood purification treatment is displayed.
Fig. 12 is a schematic diagram for describing a method of extraction performed by an extracting unit included in the blood purification system.
Fig. 13 is a schematic diagram for describing a method of extraction performed by the extracting unit included in the blood purification system.
Fig. 14 is a block diagram of a blood purification system according to a third embodiment of the present invention.
Fig. 15 is a schematic diagram illustrating items displayed on a display unit of the blood purification system.
Fig. 16 is a schematic diagram illustrating the items displayed on the display unit of the blood purification system and a list controlled to be displayed by a display control unit.
Fig. 17 is a schematic diagram illustrating, among the items displayed in the blood purification system, particular incidents occurred during the current session of blood purification treatment.
Fig. 18 is a schematic diagram illustrating items displayed when any of the items in the list is specified in the blood purification system.

### Description of Embodiments

Embodiments of the present invention will now be described specifically with reference to the drawings.

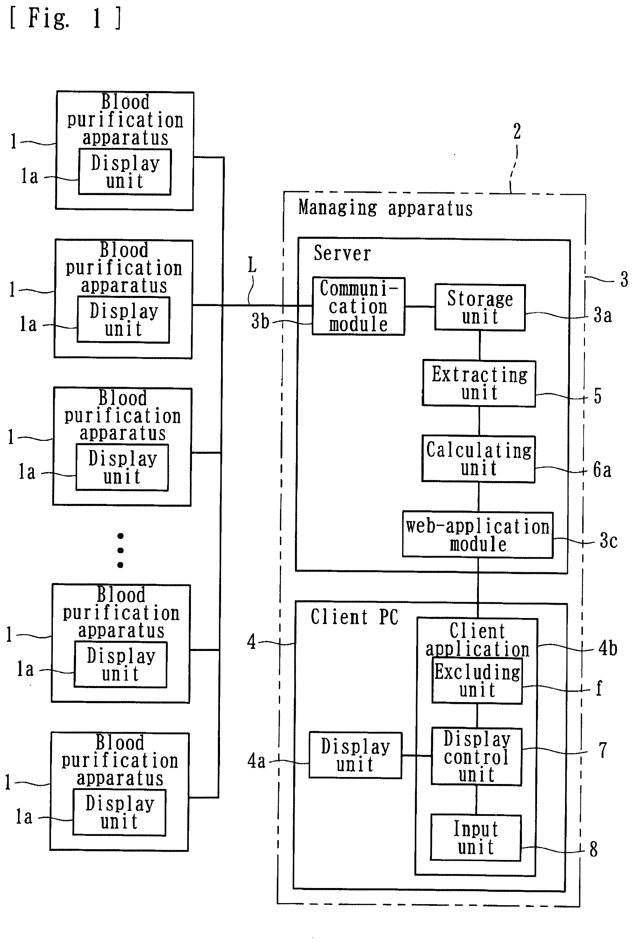

A blood purification system according to a first embodiment is capable of giving dialysis treatment as blood purification treatment to patients and includes, as illustrated in Fig. 1, blood purification apparatuses 1 and a managing apparatus 2. The blood purification apparatuses 1 and the managing apparatus 2 are electrically connected to each other through LAN cables L. Hence, the blood purification apparatuses 1 and the managing apparatus 2 can communicate with each other in such a manner as to transmit and receive information on the blood purification treatment to and from each other.

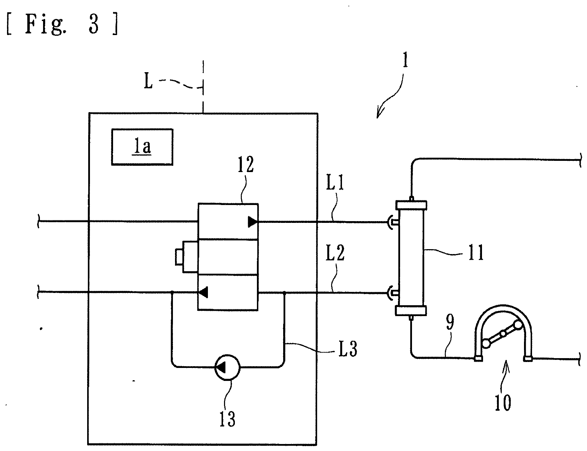

As illustrated in Fig. 3, each of the blood purification apparatuses 1 includes a dialysate introduction line L1 for introducing dialysate, a dialysate drain line L2 through which waste dialysate is drained, and a duplex pump 12 provided over the dialysate introduction line L1 and the dialysate drain line L2. A dialyzer 11 (a blood purifier) is connected to the dialysate introduction line L1 and to the dialysate drain line L2. A blood circuit 9 through which blood of a patient is caused to extracorporeally circulate is connected to the dialyzer 11. When a blood pump 10 is activated, the blood of the patient is caused to extracorporeally circulate through the blood circuit 9 and can be purified by the dialyzer 11.

A pump chamber of the duplex pump 12 is divided by a single plunger, not illustrated, into a delivery-side pump chamber connected to the dialysate introduction line L1 and a drain-side pump chamber connected to the dialysate drain line L2. When the plunger undergoes a reciprocal motion, the dialysate or a cleaning solution delivered to the delivery-side pump chamber is supplied to the dialyzer 11, while the dialysate in the dialyzer 11 is taken into the drain-side pump chamber.

The dialysate drain line L2 is provided with a bypass line L3 that bypasses the duplex pump 12. The bypass line L3 is provided with an ultrafiltration pump 13 at a halfway position thereof. When the ultrafiltration pump 13 is activated, the blood of the patient that is flowing in the dialyzer 11 can be ultrafiltered. The duplex pump 12 may be replaced with a device of a so-called chamber type.

The blood purification apparatus 1 further includes a display unit 1a and various treatment units (for example, the blood pump, an infusion pump, a syringe pump, and so forth) related to blood purification treatment (hemodialysis treatment). The treatment units are not limited to actuators such as pumps and may include various general units intended for blood purification treatment: namely, clamping units such as electromagnetic valves, and monitoring units for monitoring hydraulic pressure and so forth.

The display unit 1a is capable of displaying predetermined information on the blood purification treatment (hemodialysis treatment) and accepts predetermined input. The display unit 1a according to the present embodiment is a touch panel that accepts predetermined input made by touching on a corresponding one of positions of a screen thereof. The information on the blood purification treatment that is displayed on the display unit 1a includes a setting value representing the flow rate of the blood pump 10, the ultrafiltration pump 13, the infusion pump, or the like; the venous pressure detected by a venous pressure sensor; and the hematocrit value or the like detected by a hematocrit sensor or the like.

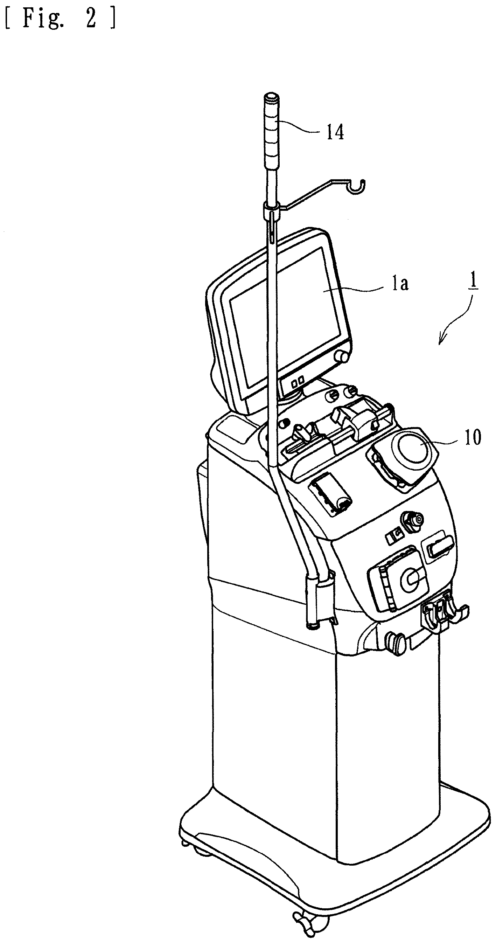

The blood purification apparatus 1 further includes a speaker capable of generating any type of output (output such as a voice or a sound effect). The speaker makes it possible to generate an alarm or a warning for notifying anyone nearby of the occurrence of an abnormality in any of the devices operating for the blood purification treatment or in the patient, and to give the operator a guidance, with a voice or the like, on how to handle the blood purification apparatus or how to make various settings. The blood purification apparatus 1 further includes an external indicator lamp 14 (see Fig. 2). The external indicator lamp 14 is turned on or is made to blink so that anybody nearby can notice the warning or the like more assuredly.

The managing apparatus 2 includes a server 3 and a client PC 4. The server 3 includes a communication module 3b for communication with the blood purification apparatuses 1 through the LAN cables L, a storage unit 3a serving as a database in which data is storable, a web-application module 3c that activates a client application 4b included in the client PC 4, an extracting unit 5, and a calculating unit 6a. The client PC 4 is provided in a treatment room in which the blood purification apparatuses 1 are installed. The server 3 is provided in another room (such as a computer room) separate from the treatment room.

The storage unit 3a is capable of communicating with each of the blood purification apparatuses 1 through the communication module 3b. The storage unit 3a is also capable of storing information on the blood purification treatment in a time course with the progress of the treatment and storing the information as a history for each treatment session. For example, information transmitted from the managing apparatus 2 to each blood purification apparatus 1 (information required for the current treatment session) includes setting information on the blood purification apparatus 1, master information that enables selection of a below-described particular incident that has occurred unsteadily, personal information (such as dry weight (DW), medication instructions, and test values) on the patient to be treated, and so forth.

Information (the history) transmitted from each blood purification apparatus 1 to the managing apparatus 2 includes monitoring information on the blood purification apparatus 1, warnings and notifications generated by the blood purification apparatus 1, a record of actions including a log of operations performed in response to the warnings and notifications, self-diagnostic information on the blood purification apparatus 1, information on the patient's blood pressure and pulse measured by a blood pressure monitor provided to the blood purification apparatus 1, information on the patient's temperature inputted to the blood purification apparatus 1 by the operator, identification codes selected from the master information by the medical worker, and so forth.

The history includes particular incidents that have occurred unsteadily (suddenly) during the blood purification treatment. The particular incidents include, for example, unsteady incidents related to the patient's behavior during the blood purification treatment (events such as food taking, the release of the patient for a bathroom break, and a change in body position; sudden incidents such as dislodgement of a puncture needle; and the like) or unsteady incidents related to the patient's condition (a drop in blood pressure, complaints such as feelings of discomfort and itching, and the like), and unsteady incidents occurring in the apparatus during the blood purification treatment (actions taken in response to warnings and notifications generated by the blood purification apparatus 1, and the like).

The storage unit 3a is capable of associating various pieces of information with one another and storing the pieces of information as a history for each treatment session. The pieces of information to be associated with one another include, in addition to the particular incidents occurred unsteadily, the name of the patient treated, the place of treatment, the blood purification apparatus used, information transmitted to and received from the blood purification apparatus 1 during the treatment, actual values such as the patient's body weights before and after the treatment and the volume and rate of ultrafiltration, treatment conditions, the record of medicines given during the treatment, the record of medical materials used for the treatment, the record of care, and so forth.

Any particular incident can be inputted during the blood purification treatment by selecting the kind thereof (whether it is an event during the treatment, a complaint from the patient, an action taken, or the like) through the display unit 1a (the touch panel) of the blood purification apparatus 1 or through an input unit 8 included in the client application 4b. In the present embodiment, the particular incidents are mastered with identification codes that are classified by kind. If a medical worker wants to input a certain particular incident, the medical worker can select, by inputting, an identification code corresponding to the particular incident of interest and then input the actual particular incident. Hence, the storage unit 3a can store particular incidents on the basis of the identification codes.

The warnings and notifications generated by the blood purification apparatus 1 during the blood purification treatment and the record of actions including the log of operations performed in response to the warnings and notifications are also mastered with identification codes that are classified by kind. If a medical worker wants to input a certain particular incident, the medical worker can select, by inputting, an identification code corresponding to the particular incident of interest and then input the actual particular incident.

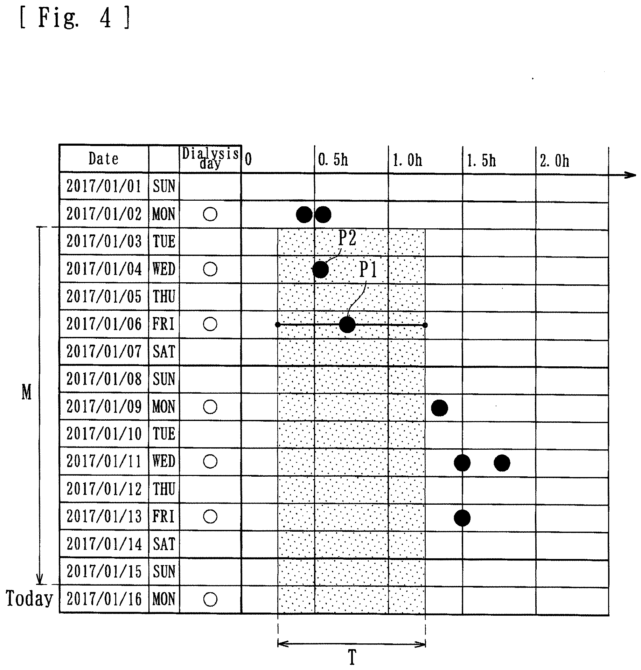

Thus, as illustrated in Figs. 4 to 6, the storage unit 3a can store the information on the blood purification treatment in a time course with the progress of the treatment (for example, sequentially from when the patient enters the dialysis room until the patient leaves the dialysis room after the completion of the treatment) and can store the information as a history for each treatment session (each dialysis date). Furthermore, the storage unit 3a can store the times of occurrence of particular incidents on the basis of their kinds and in correspondence with the time in the history. Hence, the storage unit 3a can store what kind of particular incident has occurred after how many minutes from the start of a particular one of the past treatment sessions.

The extracting unit 5 is capable of searching the histories stored in the storage unit 3a except those excluded by an excluding unit f and extracting a desired one of the particular incidents. When a desired particular incident that needs to be grasped by the medical worker is selected (particular incidents of different kinds are also selectable) and is inputted (specifically, when an identification code corresponding to a desired particular incident is inputted) through the display unit 1a or the input unit 8, the extracting unit 5 according to the present embodiment can search the histories stored in the storage unit 3a except those excluded by the excluding unit f and can extract the selected particular incident. The number of particular incidents to be selected by the medical worker is not limited to one, and a plurality of kinds of particular incidents may be selected and inputted. The kinds of particular incidents to be paid attention to are different for different patients. Therefore, the extraction of such particular incidents by the extracting unit 5 may be performed patient by patient.

The present embodiment concerns a case where the range from which the extracting unit 5 extracts a particular incident is limited to the histories of the patient to whom the blood purification treatment is going to be given. Alternatively, the extracting unit 5 may extract a particular incident from a range including histories of other patients. In the latter case, the other patients acceptable as additional conditions include patients having a similar symptom, patients of the same sex, patients of the same or a close age, patients having the same or a similar primary disease, and patients having the same or a close DW (dry weight) or test value. In addition, the medical worker may arbitrarily include or exclude other patients into or from the object of extraction by the extracting unit 5. That is, the medical worker may select patients to be included as the object of extraction for each particular incident.

A particular incident extracted as above is presented as illustrated in Figs. 4 to 6 in a time course for each of past treatment sessions. Hence, with what timings the particular incident has occurred in the past treatment sessions (dialysis dates) can be grasped. The tables illustrated in the drawings each include lines of dialysis dates arranged vertically in order and columns of treatment time slots (hours) arranged horizontally, with points (dots) of the particular incident of interest plotted with reference to time. The drawings illustrate the result of extraction of a single particular incident. If a plurality of particular incidents are extracted, the result is presented for each of the different kinds of particular incidents in a time course for each of past treatment sessions.

The calculating unit 6a is capable of calculating a time when the particular incident extracted by the extracting unit 5 occurs a predetermined or more number of times as a frequent-occurrence time slot. For example, as illustrated in Fig. 4, a period M is set as an object of calculation. Furthermore, a time slot including slots before and after a particular-incident point P1 among several particular-incident points extracted within the period M is defined as a scanning time slot T. Then, whether the number of particular-incident points extracted within the scanning time slot T is greater than or equal to a predetermined value, i.e. a threshold, is checked. The threshold may be set arbitrarily. For example, if the threshold is set to 3 and the number of particular-incident points extracted within the scanning time slot T is 2 (two particular-incident points P1 and P2) as illustrated in the drawing, it is not determined that the occurrence of the particular incident is frequent. The period M as the object of calculation can be set arbitrarily by the medical worker or the like and may be set to an indefinite value.

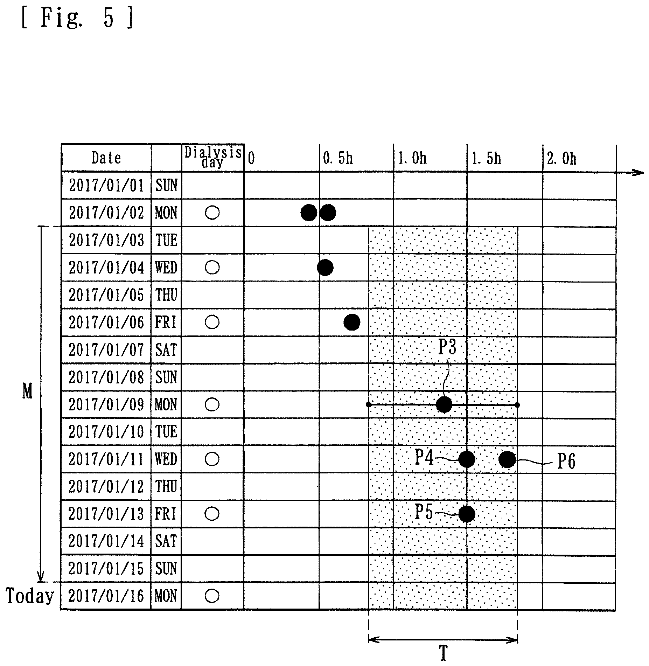

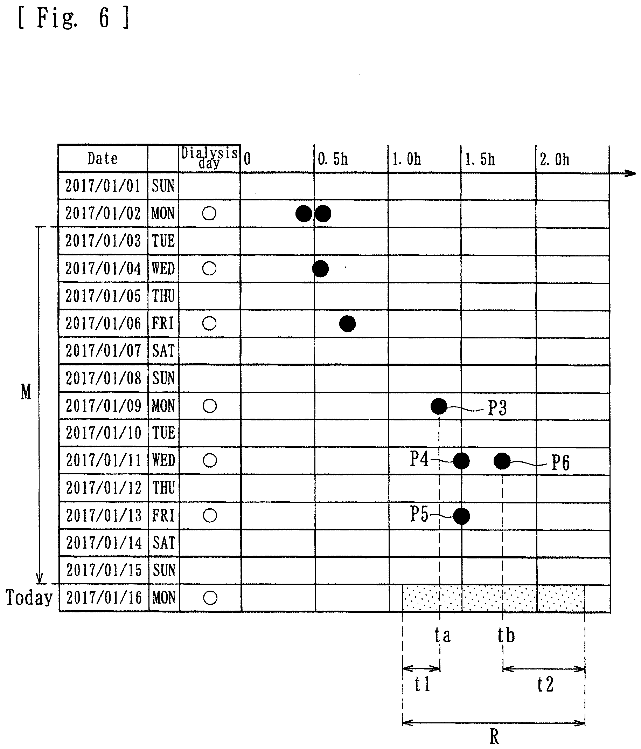

Fig. 5 illustrates a case where a time slot including slots before and after a particular-incident point P3 among several particular-incident points extracted is defined as the scanning time slot T, and the number of particular-incident points extracted within the scanning time slot T is 4 (four particular-incident points P3 to P6). In such a case, it is determined that the occurrence of the particular incident is frequent. Furthermore, as illustrated in Fig. 6, a time between a point before a point ta by an allowance t1 and a point after a point tb by an allowance t2 is calculated as a frequent-occurrence time slot R. The point ta corresponds to the particular-incident point P3 as the earliest occurrence in a time course of the treatment session. The point tb corresponds to the particular-incident point P6 as the latest occurrence in a time course of the treatment session.

The client PC 4 is a personal computer and includes a display unit 4a and the client application 4b. The display unit 4a is a liquid-crystal screen or a touch panel that is capable of displaying information on the blood purification treatment. The client application 4b is connected to the web-application module 3c of the server 3, whereby information on the blood purification treatment can be inputted thereto and outputted therefrom. The client application 4b includes the excluding unit f, a display control unit 7 and the input unit 8.

The input unit 8 accepts selective input of any of the plurality of particular incidents (for example, particular incidents of different kinds such as unsteady incidents related to the patient's behavior during the blood purification treatment or unsteady incidents related to the patient's condition, and unsteady incidents occurring in the apparatus during the blood purification treatment). In the present embodiment, the frequent-occurrence time slot of a particular incident inputted through the input unit 8 can be calculated by the calculating unit 6a and can be controlled to be displayed by the display control unit 7. The input may be made through the display unit 1a (a touch panel or the like) of the blood purification apparatus 1, instead of the input unit 8.

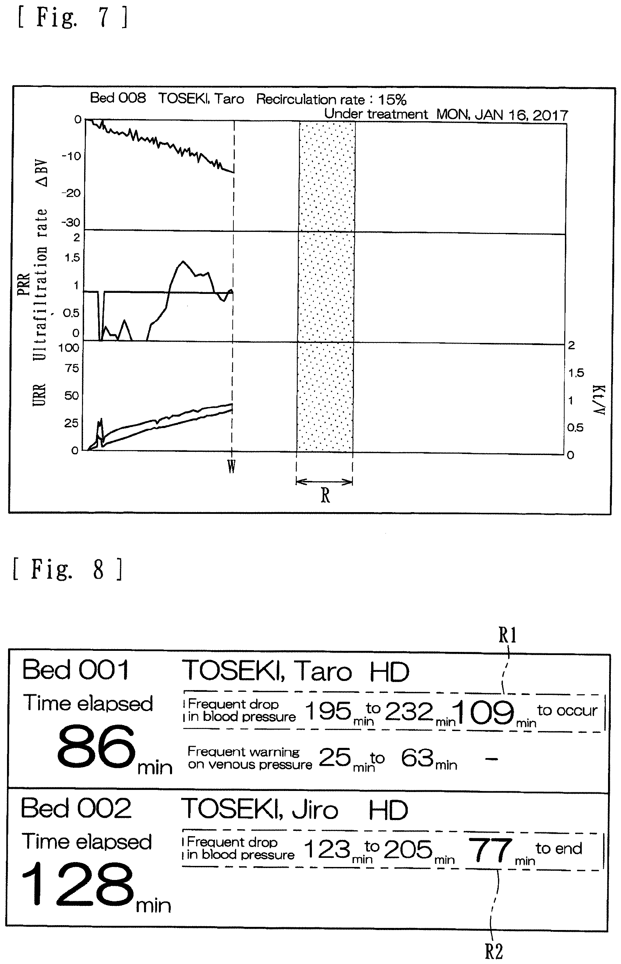

The display control unit 7 is capable of controlling various pieces of information based on the history extracted by the extracting unit 5 to be displayed on the display unit 4a. In the present embodiment, the display control unit 7 is capable of controlling the frequent-occurrence time slot calculated by the calculating unit 6a to be displayed on the display unit 4a during the current session of blood purification treatment. Specifically, as illustrated in Fig. 7, the display control unit 7 according to the present embodiment is capable of controlling pieces of information on the current session of blood purification treatment to be displayed as graphs with the progress of time (the case illustrated in the drawing includes, with the horizontal axis representing the time axis, a line graph of ΔBV in an upper part, line graphs of PRR and ultrafiltration rate in a middle part, and line graphs of URR and Kt/V in a lower part), and is also capable of controlling the frequent-occurrence time slot R calculated by the calculating unit 6a to be displayed with reference to the time axis of the graphs.

In particular, as illustrated in the drawing, the display control unit 7 according to the present embodiment is capable of controlling the frequent-occurrence time slot R calculated by the calculating unit 6a to be displayed as a band represented with reference to the time axis (the time axis of the current session of blood purification treatment). Since the frequent-occurrence time slot R is displayed as a band as described above, the start time and the end time of the frequent-occurrence time slot R can be grasped simultaneously and visually. Furthermore, about how much time is left from a current time W before the frequent-occurrence time slot R is reached can be grasped visually.

The display control unit 7 according to the present embodiment is capable of controlling information on the current session of blood purification treatment to be displayed as graphs with the progress of time, and is also capable of controlling the frequent-occurrence time slot R calculated by the calculating unit 6a to be displayed as a band represented with reference to the time axis of the graphs. Alternatively, as illustrated in Fig. 8, the display control unit 7 may be capable of controlling the frequent-occurrence time slot (R1 or R2) calculated by the calculating unit 6a to be displayed numerically on the display unit 4a during the current session of blood purification treatment. In the latter case, the frequent-occurrence time slot (R1 or R2) is displayed for each of a particular incident of "drop in blood pressure" and a particular incident of "warning on venous pressure". The frequent-occurrence time slot may be displayed for any other particular incident in any other form.

If a plurality of particular incidents are inputted through the input unit 8 (or the display unit 1 a), the display control unit 7 may control respective frequent-occurrence time slots to be displayed such that the kinds of those particular incidents are identifiable. The kinds of the particular incidents can be made identifiable by displaying different frequent-occurrence time slots (represented as graphs or the like) in different colors (including different tones). Moreover, the display control unit 7 may control the frequent-occurrence time slots to be displayed such that the frequencies of occurrence of the respective particular incidents are identifiable. In such a case, frequent-occurrence time slots of higher frequencies and frequent-occurrence time slots of lower frequencies can be made identifiable for each of the particular incidents by displaying the frequent-occurrence time slots (represented as graphs or the like) of different frequencies in different colors (including different tones).

The extraction of a particular incident by the extracting unit 5 and the calculation of the frequent-occurrence time slot by the calculating unit 6a are preferably performed when the particular incident inputted through the input unit 8 is changed, when the recording of the patient's history for each treatment session is completed and determined, or in midnight hours or the like during which the blood purification system is out of operation. Needless to say, if a client PC 4 operating at an extremely high processing speed is employed, the extraction of a particular incident by the extracting unit 5 and the calculation of the frequent-occurrence time slot by the calculating unit 6a may be performed immediately before the blood purification treatment is started.

The client application 4b of the managing apparatus 2 according to the present embodiment includes the excluding unit f. The excluding unit f is capable of excluding any of the histories stored in the storage unit 3a from the object of search to be performed by the extracting unit 5. Hence, the extracting unit 5 according to the present embodiment can extract any particular incident by searching histories stored in the storage unit 3a except those excluded by the excluding unit f.

The excluding unit f according to the present embodiment allows the medical worker or the like to select and specify any item to be excluded from the object of search (i.e. from the histories stored in the storage unit 3a) by, for example, checking or unchecking relevant checkboxes on the client application 4b. Exemplary items to be excluded by the excluding unit f include histories except those of the current patient who is going to take blood purification treatment, patients other than the current patient and of a different sex or different ages, patients not having the same or a similar primary disease, patients not having the same or a close DW (dry weight) or test value, and so forth.

The excluding unit f may be configured such that items to be excluded from the object of search performed by the extracting unit 5 are specified in units of one history. In such a case, the entirety of the history of a single treatment session can be excluded from the object of search. Hence, information that is unfavorable for performing highly accurate extraction can be excluded in units of one treatment session. Furthermore, the excluding unit f is configured such that items to be excluded from the object of search are specified in units of one piece of data included in each of the histories (in the present embodiment, in units of one kind of particular incident). In such a case, the object of exclusion can be specified more precisely. In addition to the exclusion in units of one piece of data, exclusion in units of one treatment date or the like is also possible. In such a case, the object of exclusion may be specified on a matrix screen.

According to the present embodiment, the extracting unit 5 is capable of extracting a particular incident by searching the histories stored in the storage unit 3a except those excluded by the excluding unit f. Furthermore, the blood purification system includes the calculating unit 6a capable of calculating a time when the particular incident extracted by the extracting unit 5 occurs a predetermined or more number of times as the frequent-occurrence time slot R. Furthermore, the display control unit 7 is capable of controlling the frequent-occurrence time slot calculated by the calculating unit 6a to be displayed on the display unit during the current session of blood purification treatment. Therefore, history search suitable for individual purposes of extraction is realized, whereby the reliability of information to be extracted and displayed can be increased. Furthermore, the medical worker can grasp the frequent-occurrence time slot R of the particular incident in advance.

Thus, the timing of frequent occurrence (the frequent-occurrence time slot) of a desired particular incident that is considered to be paid attention to by the medical worker can be grasped in advance during the current session of blood purification treatment. Therefore, preparations and confirmations for the particular incident of interest can be made in advance, and an action in response to the occurrence of the particular incident can be taken quickly and appropriately. Furthermore, since the frequent-occurrence time slots of particular incidents can be summarized automatically, not manually by the operator, the number of steps of operation can be reduced. Moreover, since factors such as the threshold for the frequent-occurrence time slot and the scanning time slot T can be set arbitrarily on the basis of viewpoints of experienced and high-skill medical workers, even unexperienced and low-skill medical workers can grasp the frequent-occurrence time slots of the particular incidents to be paid attention to.

Now, a second embodiment (not forming part of the invention) will be described.

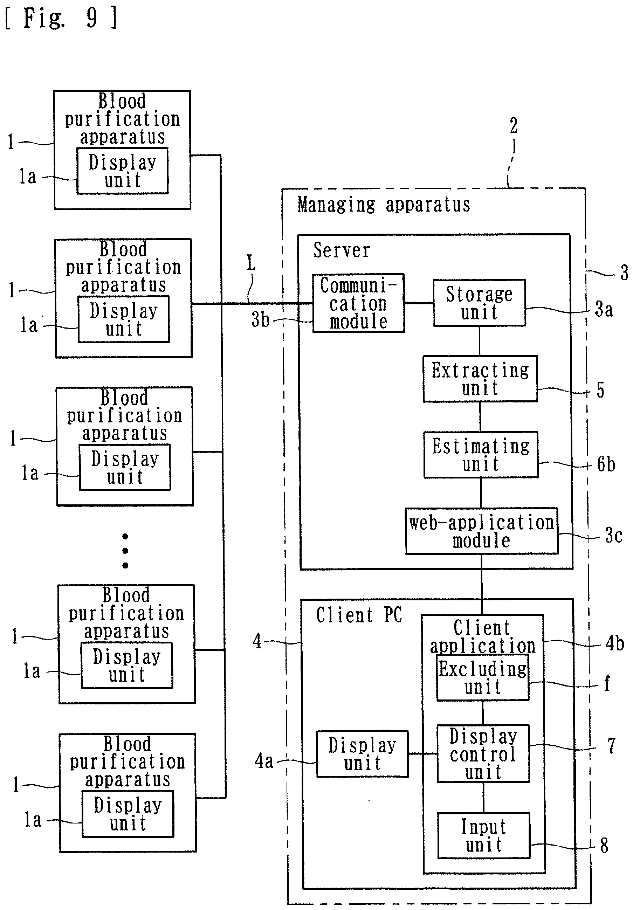

As with the case of the first embodiment, a blood purification system according to the present embodiment is capable of giving dialysis treatment as blood purification treatment to patients and includes, as illustrated in Fig. 9, blood purification apparatuses 1 and a managing apparatus 2. The blood purification apparatuses 1 and the managing apparatus 2 are electrically connected to each other through LAN cables L. Hence, the blood purification apparatuses 1 and the managing apparatus 2 can communicate with each other in such a manner as to transmit and receive information on the blood purification treatment to and from each other. Elements that are the same as those described in the first embodiment are denoted by corresponding ones of the reference numerals, and detailed description of such elements is omitted.

The extracting unit 5 according to the present embodiment is capable of searching the histories stored in the storage unit 3a except those excluded by the excluding unit f and extracting, as an approximate history, a history including a particular parameter of interest approximate to that observed at the current time in the current session of blood purification treatment. When a particular parameter that needs to be grasped by the medical worker is selected (particular parameters of different kinds are also selectable) and is inputted (specifically, when an identification code corresponding to a desired particular parameter is inputted) through the display unit 1a or the input unit 8, the extracting unit 5 according to the present embodiment can search the histories stored in the storage unit 3a except those excluded by the excluding unit f and can extract a particular parameter that exhibits an approximate transition.

A specific method of extraction by the extracting unit 5 is as follows, for example. When a particular parameter (such as Kt/V or the like) desired to estimate and conditions relevant thereto are inputted through the input unit 8 or the like with a predetermined timing during the current session of blood purification treatment, the extracting unit 5 searches for a history (from the histories except those excluded by the excluding unit f) exhibiting an approximate value within a predetermined time range (a preset period) starting from the current time. The range (scanning range) for which judgement of approximation is to be made is set in advance for each of different kinds of particular parameters. For example, a period between a point where the value of the particular parameter is greater than the current value of the parameter by a predetermined amount (i.e. the upper limit of the threshold for scanning) and a point where the value of the particular parameter is smaller than the current value of the parameter by a predetermined amount (i.e. the lower limit of the threshold for scanning) may be set in advance as the scanning range.

Alternatively, the entire period of each of the histories stored in the storage unit 3a except any period excluded by the excluding unit f may be set as the range of search, or the range to be searched may be made specifiable. When a particular parameter (such as Kt/V or the like) desired to estimate and conditions relevant thereto are inputted through the input unit 8 or the like with a predetermined timing during the treatment, any of all histories within the above scanning range starting from the current time can be extracted as an approximate history.

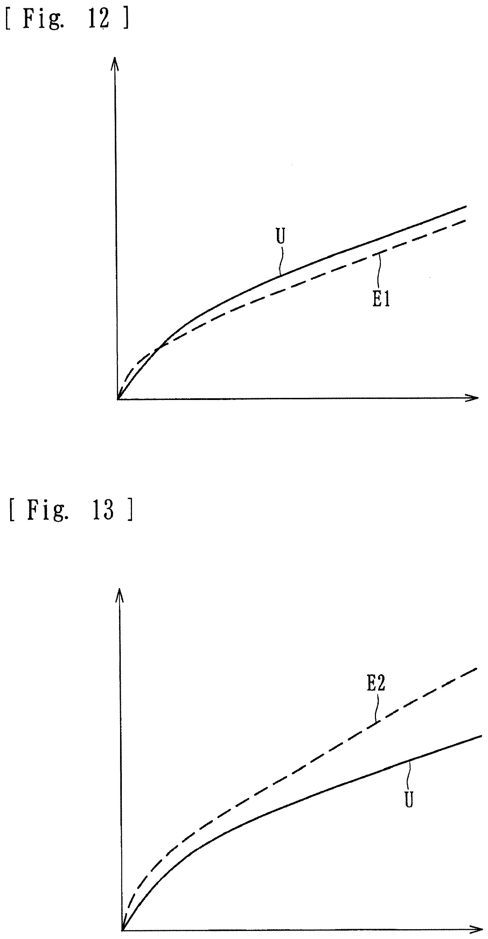

As another method of extraction, the extracting unit 5 may be capable of searching the histories stored in the storage unit 3a except those excluded by the excluding unit f and extracting, as an approximate history, a history including the particular parameter of interest exhibiting a transition approximate to that observed until the current time in the current session of blood purification treatment. In such a case, as illustrated in Figs. 12 and 13, a transition of a particular parameter U observed until the current time in the current session of blood purification treatment and a transition of the particular parameter (E1 or E2) in a specified one of the histories stored in the storage unit 3a are superposed on each other, and the area of difference between the two is calculated.

For example, the extracting unit 5 may be configured as follows. If the area of difference between the transition of the particular parameter U observed until the current time and the transition of the particular parameter E1 stored as the history (see Fig. 12) does not exceed a predetermined threshold, the particular parameter E1 is extracted as an approximate history. If the area of difference between the transition of the particular parameter U observed until the current time and the transition of the particular parameter E2 stored as the history (see Fig. 13) exceeds the predetermined threshold, the particular parameter E2 is not extracted as an approximate history.

The present embodiment concerns a case where the range from which the extracting unit 5 extracts a particular parameter is limited to the histories of the patient to whom the blood purification treatment is going to be given. Alternatively, the extracting unit 5 may extract a particular parameter from a range including histories of other patients. In the latter case, the other patients acceptable as additional conditions include patients having a similar symptom, patients of the same sex, patients of the same or a close age, patients having the same or a similar primary disease, and patients having the same or a close DW (dry weight) or test value. In addition, the medical worker may arbitrarily include or exclude other patients into or from the object of extraction by the extracting unit 5. That is, the medical worker may select patients to be included as the object of extraction for each particular incident.

An estimating unit 6b estimates a transition of a particular parameter to be observed after the current time in the current session of blood purification treatment from the approximate history extracted by the extracting unit 5. Specifically, the estimating unit 6b according to the present embodiment is capable of analyzing all approximate histories extracted by the extracting unit 5, calculating upper limits and lower limits for respective time slots, and estimating as an estimated upper-limit transition by connecting the upper limits to one another and as an estimated lower-limit transition by connecting the lower limits to one another.

More specifically, if all of the extracted approximate histories are graphed with one specific time axis, the greatest value and the smallest value at every point in time can be identified. Accordingly, a graph as a series of the greatest values and a graph as a series of the smallest values are obtained. The transition of the upper limit and the transition of the lower limit that are obtained as above are estimated as a transition of the particular parameter to be observed after the current time in the current session of blood purification treatment. Such a transition of a particular parameter to be observed after the current time may alternatively be estimated by any other mathematical or graphical method.

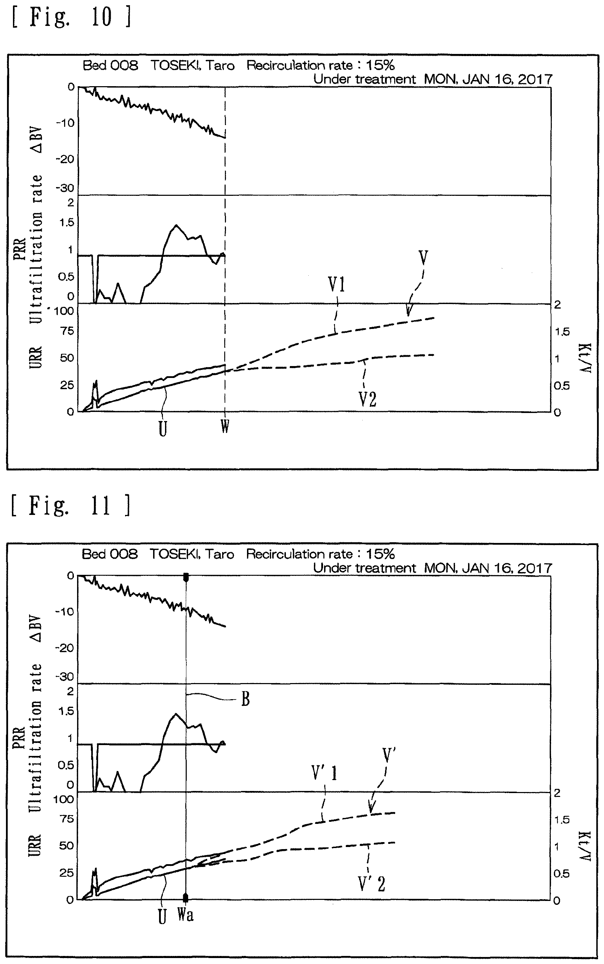

The display control unit 7 is capable of controlling the transition of the particular parameter estimated by the estimating unit 6b and the transition of the particular parameter observed during the current session of blood purification treatment to be displayed simultaneously on the display unit 4a. Specifically, as illustrated in Fig. 10, the display control unit 7 according to the present embodiment is capable of controlling a particular parameter as information on the current session of blood purification treatment to be displayed as a graph with the progress of time (the case illustrated in the drawing includes, with the horizontal axis representing the time axis, a line graph of ΔBV in an upper part, line graphs of PRR and ultrafiltration rate in a middle part, and line graphs of URR and Kt/V in a lower part), and is also capable of controlling a transition V of the particular parameter estimated by the estimating unit 6b to be displayed with reference to the time axis of the graph.

That is, as illustrated in the drawing, the transition of Kt/V observed during the current session of blood purification treatment is displayed as a transition of a particular parameter. Simultaneously, with a current time W in the graph being defined as the start point, a transition of the particular parameter (Kt/V in this case) estimated by the estimating unit 6b is displayed. In particular, as illustrated in the drawing, the display control unit 7 according to the present embodiment controls an upper-limit transition V1 and a lower-limit transition V2 of the particular parameter that are estimated by the estimating unit 6b to be displayed as graphs such that the transition V (including the upper-limit transition V1 and the lower-limit transition V2) of the particular parameter estimated by the estimating unit 6b is distinguishable (in the present embodiment, the transition V is represented by dotted lines).

Furthermore, in the present embodiment, a past time before the current time W in the current session of blood purification treatment is specifiable through the input unit 8. Therefore, the extracting unit 5 can search the histories except those excluded by the excluding unit f for any history including the particular parameter of interest approximate to that observed at the point inputted through the input unit 8, and can then extract that history as an approximate history. For example, as illustrated in Fig. 11, if a time-shift bar B movable in the graph with an operation on the input unit 8 is displayed, a past time Wa before the current time W can be specified.

When a past time Wa is specified by using the time-shift bar B, the extracting unit 5 searches for any history including the particular parameter of interest approximate to that observed at the past time Wa and extracts that history as an approximate history. Then, the estimating unit 6b estimates a transition of the particular parameter to be observed after the time Wa from the extracted approximate history. A transition V' of the particular parameter estimated as above by the estimating unit 6b is displayed as a graph starting from the specified past time Wa. In such a case as well, the transition V' of the particular parameter estimated by the estimating unit 6b includes an upper-limit transition V'1 and a lower-limit transition V'2, which are displayed in such a manner as to be distinguishable (in the present embodiment, represented by dotted lines) from the transition U of the particular parameter.

The display control unit 7 according to the present embodiment is capable of controlling the information on the current session of blood purification treatment to be displayed as a graph (the transition U) with the progress of time, and the transition (V or V') of the particular parameter estimated by the estimating unit 6b to be displayed as the upper-limit transition (V1 or V'1) and the lower-limit transition (V2 or V'2). Alternatively, the information may be displayed in any other form (for example, as a transition of the average value or the like).

The extraction of an approximate history by the extracting unit 5 and the estimation of the transition of a particular parameter by the estimating unit 6b are preferably performed when a particular parameter desired to estimate is specified through the input unit 8, or when a past time Wa is specified with the time-shift bar B operated through the input unit 8. While the present embodiment concerns a case where Kt/V is specified as the particular parameter, another particular parameter may be specified through the input unit 8 so that the transition of that particular parameter can be estimated. Furthermore, while the present embodiment concerns a case where the particular parameter estimated by the estimating unit 6b is made distinguishable by being displayed as a dotted line, the estimated particular parameter may be made distinguishable in any other way, for example, by being displayed in a different color.

According to the above embodiment, the extracting unit 5 is capable of searching the histories stored in the storage unit 3a except those excluded by the excluding unit f and extracting, as an approximate history, a history including the particular parameter approximate to the particular parameter observed at the current time in the current session of blood purification treatment or a history including the particular parameter exhibiting a transition approximate to the transition of the particular parameter observed until the current time in the current session of blood purification treatment. Furthermore, the blood purification system includes the estimating unit 6b that estimates a transition of the particular parameter to be observed after the current time in the current session of blood purification treatment from the approximate history extracted by the extracting unit 5. Furthermore, the display control unit 7 is capable of controlling the transition of the particular parameter estimated by the estimating unit 6b and the transition of the particular parameter observed in the current session of blood purification treatment to be displayed simultaneously on the display unit. Therefore, history search suitable for individual purposes of extraction is realized, whereby the reliability of information to be extracted and displayed can be increased. Furthermore, whether the current transition of the particular parameter is normal when compared with the transition of the particular parameter estimated from the histories can be grasped quickly and accurately.

Now, a third embodiment of the present invention will be described.

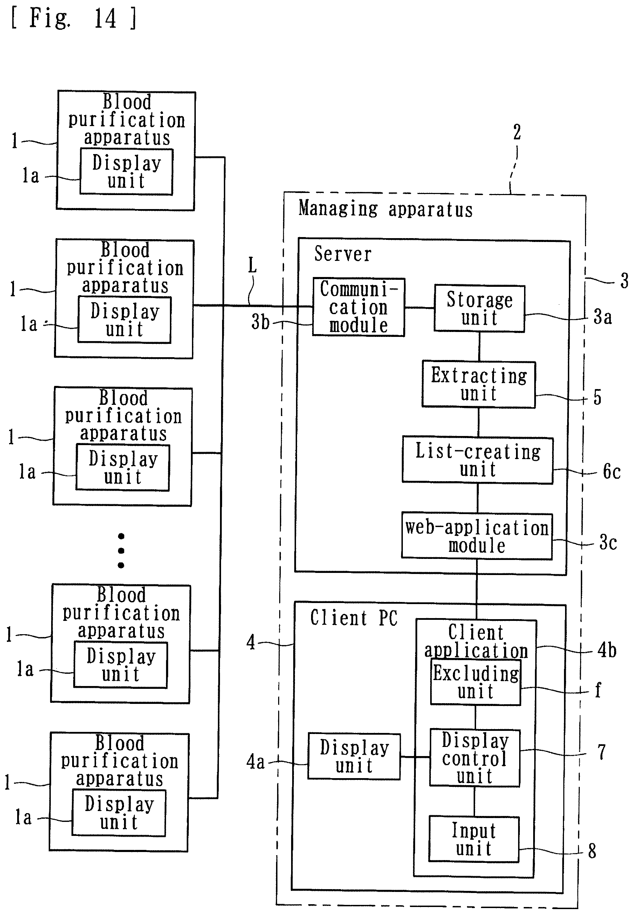

As with the cases of the first and second embodiments, a blood purification system according to the present embodiment is capable of giving dialysis treatment as blood purification treatment to patients and includes, as illustrated in Fig. 14, blood purification apparatuses 1 and a managing apparatus 2. The blood purification apparatuses 1 and the managing apparatus 2 are electrically connected to each other through LAN cables L. Hence, the blood purification apparatuses 1 and the managing apparatus 2 can communicate with each other in such a manner as to transmit and receive information on the blood purification treatment to and from each other. Elements that are the same as those described in the first and second embodiments are denoted by corresponding ones of the reference numerals, and detailed description of such elements is omitted.

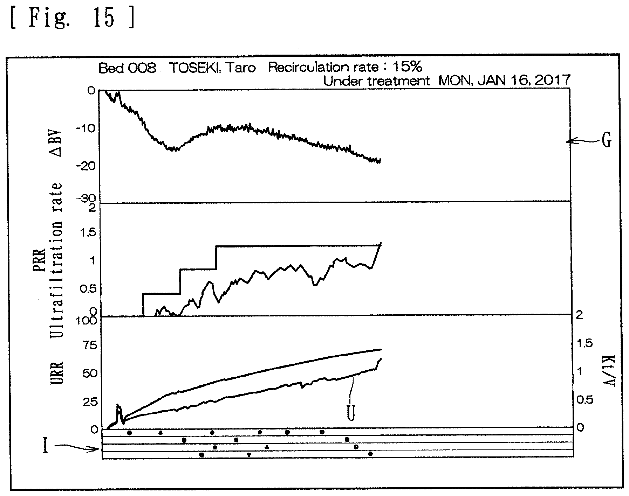

As illustrated in Fig. 15, for example, the display control unit 7 according to the present embodiment is capable of controlling particular parameters as information on the current session of blood purification treatment to be displayed as a graph G with the progress of time during the blood purification treatment (the case illustrated in the drawing includes, with the horizontal axis representing the time axis, a line graph of ΔBV in an upper part, line graphs of PRR and ultrafiltration rate in a middle part, and line graphs of URR and Kt/V in a lower part), and also controls an event bar I to be displayed below the graph G.

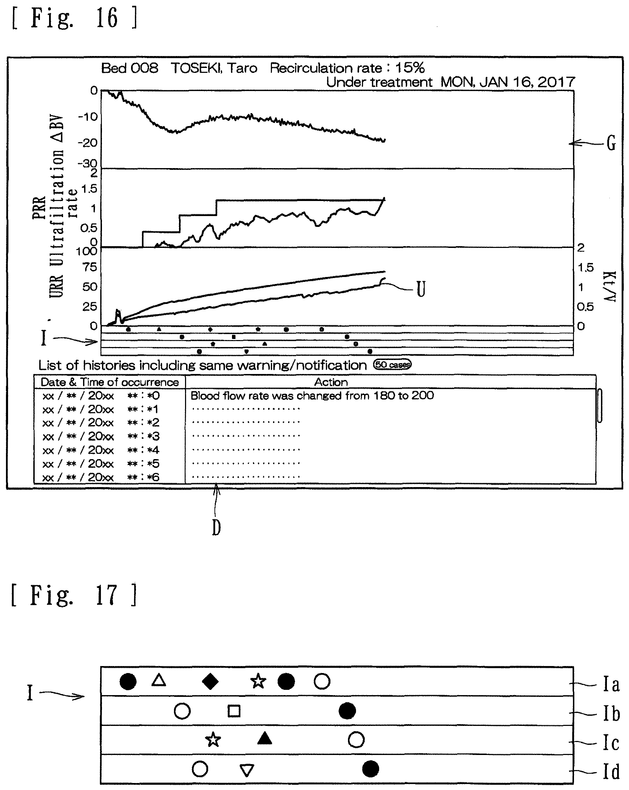

As illustrated in Fig. 17, the event bar I illustrates particular incidents having occurred unsteadily during the current session of blood purification treatment such that the kinds thereof are identifiable. Any of the illustrated particular incidents is selectable by its kind. For example, the event bar I includes, in order from the top, a first line (the top line) la for displaying particular incidents such as warnings or notifications generated by the blood purification apparatus 1 and the log of operations performed in response to the warnings or notifications, a second line Ib for displaying particular incidents related to warnings or notifications generated when monitored data exceeds a threshold defined by the managing apparatus 2, a third line Ic for displaying particular incidents related to a change in body position of the patient, complaints from the patient, and the like that occur during the blood purification treatment, and a fourth line (the bottom line) Id for displaying particular incidents related to the record of actions taken.

If any particular incident occurs during the blood purification treatment, a symbol (such as a black dot, a white dot, a star mark, a triangular mark, or the like) corresponding to the kind of that particular incident is displayed in a corresponding one of the lines of the event bar I, whereby the kind of the particular incident can be identified. While the present embodiment concerns a case where the kind of each particular incident is identifiable by the symbol, the kind of each particular incident may be made identifiable by the color, character, or the like. Thus, not only the transition of a particular parameter observed but also a particular incident occurred during the current session of blood purification treatment can be displayed on the display unit 4a.

The input unit 8 accepts selective input of any of the plurality of particular incidents (for example, particular incidents of different kinds such as unsteady incidents related to the patient's behavior during the blood purification treatment or unsteady incidents related to the patient's condition, and unsteady incidents occurring in the apparatus during the blood purification treatment). In the present embodiment, any of the particular incidents in the event bar I displayed on the display unit 4a is selectable, and any of the particular parameters displayed on the display unit 4a is selectable. The input may be made through the display unit 1a (a touch panel or the like) of the blood purification apparatus 1, instead of the input unit 8.

The extracting unit 5 according to the present embodiment is capable of searching the histories stored in the storage unit 3a except those excluded by the excluding unit f and extracting a desired one of the particular incidents. When a desired particular incident or particular parameter that needs to be grasped by the medical worker is selected (particular incidents or particular parameters of different kinds are also selectable) and is inputted (specifically, when an identification code corresponding to a desired particular incident is inputted) through the display unit 1a or the input unit 8, the extracting unit 5 according to the present embodiment can search the histories stored in the storage unit 3a except those excluded by the excluding unit f and extract, as reference histories, a plurality of histories each including the inputted particular incident (the particular incident occurred during the current session of blood purification treatment) or a plurality of histories each including the particular parameter approximate to the inputted particular parameter (the particular parameter observed during the current session of blood purification treatment). The number of particular incidents and particular parameters to be selected by the medical worker is not limited to one, and a plurality of kinds of particular incidents and particular parameters may be selected and inputted. The kinds of particular incidents to be paid attention to are different for different patients. Therefore, the extraction of such particular incidents by the extracting unit 5 may be performed patient by patient.

The present embodiment concerns a case where the range from which the extracting unit 5 extracts a particular incident or a particular parameter is limited to the histories of the patient to whom the blood purification treatment is going to be given. Alternatively, the extracting unit 5 may extract a particular incident or a particular parameter from a range including histories of other patients. In the latter case, the other patients acceptable as additional conditions include patients having a similar symptom, patients of the same sex, patients of the same or a close age, patients having the same or a similar primary disease, and patients having the same or a close DW (dry weight) or test value. In addition, the medical worker may arbitrarily include or exclude other patients into or from the object of extraction by the extracting unit 5. That is, the medical worker may select patients to be included as the object of extraction for each particular incident.

More specifically, the object of extraction by the extracting unit 5 includes histories each including any particular incident occurred during the current session of blood purification treatment, and histories each including any particular parameter of interest approximate to that observed during the current session of blood purification treatment. Histories each including any particular incident occurred during the current session of blood purification treatment are extracted as follows, for example. When a particular incident is specified by inputting a corresponding one of symbols displayed on the event bar I, a plurality of histories each including the specified particular incident are extracted as reference histories, with the particular incident being associated with information on the time in each of the past treatment sessions. Hence, with what timing in each of the past treatment sessions (dialysis days) the particular incident has occurred can be grasped.

Histories each including any particular parameter of interest approximate to that observed during the current session of blood purification treatment are extracted as follows, for example. When a particular parameter (such as Kt/V or the like) desired to display as a list is inputted through the input unit 8 or the like (for example, when any particular parameter displayed as a graph G is specified through the input unit 8) with a predetermined timing during the current session of blood purification treatment, the extracting unit 5 searches for histories (except those excluded by the excluding unit f) each including the particular parameter exhibiting an approximate value within a predetermined time range (a preset period) starting from the current time. The range (scanning range) for which judgement of approximation is to be made is set in advance for each of different kinds of particular parameters. For example, a period between a point where the value of the particular parameter is greater than the current value of the particular parameter by a predetermined amount (i.e. the upper limit of the threshold for scanning) and a point where the value of the particular parameter is smaller than the current value of the particular parameter by a predetermined amount (i.e. the lower limit of the threshold for scanning) may be set in advance as the scanning range.

Thus, the extracting unit 5 can search for and extract, as reference histories, a plurality of histories each including any particular parameter of interest exhibiting a value approximate to that observed at the current time in the current session of blood purification treatment. Note that the entire period, except the period excluded by the excluding unit f, of each of the histories stored in the storage unit 3a may be set as the range of search, or the range to be searched may be made specifiable. When a particular parameter (such as Kt/V or the like) desired to list is inputted through the input unit 8 or the like with a predetermined timing during the treatment, any of all histories within the above scanning range starting from the current time can be extracted as histories for reference.

As another method of extraction, the extracting unit 5 may be capable of searching the histories stored in the storage unit 3a and extracting, as reference histories, histories each including the particular parameter of interest exhibiting a transition approximate to that observed until the current time in the current session of blood purification treatment. In such a case, as described in the second embodiment with reference to Figs. 12 and 13, a transition of a particular parameter U observed until the current time in the current session of blood purification treatment and a transition of the particular parameter (E1 or E2) in each of specified ones of the histories stored in the storage unit 3a are superposed on each other, and the area of difference between the two is calculated.

For example, the extracting unit 5 may be configured as follows. If the area of difference between the transition of the particular parameter U observed until the current time and the transition of the particular parameter E1 stored as the history (see Fig. 12) does not exceed a predetermined threshold, the particular parameter E1 is extracted as a reference history. If the area of difference between the transition of the particular parameter U observed until the current time and the transition of the particular parameter E2 stored as the history (see Fig. 13) exceeds the predetermined threshold, the particular parameter E2 is not extracted as a reference history.

In the present embodiment, a past time before the current time in the current session of blood purification treatment is specifiable through the input unit 8, and the extracting unit 5 is capable of searching for and extracting, as reference histories, histories each including a particular parameter of interest approximate to that observed at the time inputted through the input unit 8. Specifically, when a past time instead of the current time is specified, the extracting unit 5 can search the histories except those excluded by the excluding unit f for histories each including a particular parameter of interest approximate to that observed at the specified time, and extract the histories as reference histories.

A list-creating unit 6c is capable of creating a list of the plurality of reference histories extracted by the extracting unit 5. The list includes a plurality of lines for the respective reference histories. The lines each provide information such as treatment date, treatment time, a particular incident, and so forth. When histories each including any particular incident occurred during the current session of blood purification treatment are extracted as reference histories by the extracting unit 5, the list-creating unit 6c sets the order of the list in accordance with the degree of approximation in the timing of occurrence of the particular incident (for example, the list is created in descending order of closeness in the timing of occurrence). Furthermore, on condition that any warning or notification is generated during the current session of blood purification treatment, the extracting unit 5 may be allowed to extract, as reference histories, histories each including the warning or notification as a particular incident so that the extracted reference histories are included in the list to be created by the list-creating unit 6c.

Furthermore, when histories each including any particular parameter of interest approximate to that observed during the current session of blood purification treatment are extracted as reference histories by the extracting unit 5, the list-creating unit 6c sets the order of the list in accordance with the degree of approximation in the particular parameter (for example, the list is created in descending order of closeness in the degree of approximation). The order of the list to be displayed may be changeable in any way. For example, the order of the list may be set with a sort key composed of a plurality of parameters, or the order of display may be set initially as desired.

The display control unit 7 is capable of controlling the list created by the list-creating unit 6c to be displayed on the display unit 4a. For example, as illustrated in Fig. 16, a list D is displayed below the graph G that provides information on the current session of blood purification treatment (i.e. further below the event bar I). As described above, the list D provides a plurality of reference histories as histories each including any particular incident occurred during the current session of blood purification treatment or histories each including any particular parameter approximate to that observed during the current session of blood purification treatment.

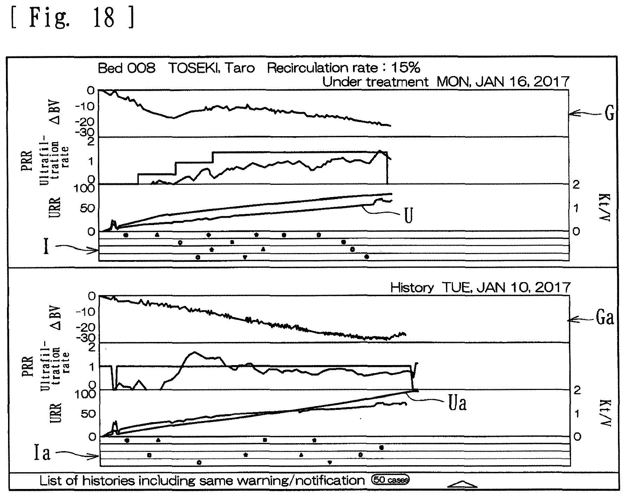

When a medical worker selects and inputs any of the reference histories displayed in the list D, a graph Ga illustrated in Fig. 18 is displayed below the graph G. The graph Ga illustrates the transition of the particular parameter in the reference history selected from the list D by the medical worker. For example, a particular parameter Ua corresponding to the particular parameter U observed during the current session of blood purification treatment is displayed.

The extraction of approximate histories by the extracting unit 5 and the creation of a list by the list-creating unit 6c are preferably performed when any particular incident or any particular parameter desired to list is specified through the input unit 8 or when a past point is specified through the input unit 8. While the present embodiment concerns a case where Kt/V is specified as the particular parameter, any other particular parameter may be specified through the input unit 8, and reference histories each including that particular parameter approximate thereto may be extracted.

According to the present embodiment, the extracting unit 5 is capable of searching the histories stored in the storage unit 3a except those excluded by the excluding unit f and extracting, as reference histories, a plurality of histories each including any particular incident having occurred during the current session of blood purification treatment or a plurality of histories each including any particular parameter approximate to that observed during the current session of blood purification treatment. Furthermore, the blood purification system includes the list-creating unit 6c capable of creating a list of the plurality of reference histories extracted by the extracting unit 5. Furthermore, the display control unit 7 is capable of controlling the list created by the list-creating unit 6c to be displayed on the display unit 4a. Therefore, history search suitable for individual purposes of extraction is realized, whereby the reliability of information to be extracted and displayed can be increased. Furthermore, an action in response to any particular incident that occurs unsteadily or any transition of a particular parameter can be taken quickly and appropriately.

According to each of the first to third embodiments, the blood purification system includes the extracting unit 5 capable of searching for and extracting any history satisfying a predetermined condition from among the histories stored in the storage unit 3a, the excluding unit f capable of excluding any of the histories stored in the storage unit 3a from the object of search performed by the extracting unit 5, and the display control unit 7 capable of controlling various pieces of information based on the history extracted by the extracting unit 5 to be displayed on the display unit 4a. Therefore, the histories stored during the blood purification treatment can be utilized effectively. Furthermore, history search suitable for individual purposes of extraction is realized, whereby the reliability of information to be extracted and displayed can be increased.

For example, if an action that is being taken in response to a warning generated by the blood purification apparatus 1 is inappropriate, the warning may be generated repeatedly. In such a case, although it should be recorded that a particular incident (generation of a warning) has occurred once, it is actually recorded in the history that the particular incident has occurred the number of times of repetition. To avoid such a situation, if the second and subsequent occurrences of the particular incident are excluded by the excluding unit f, the extraction by the extracting unit 5 can be performed accurately. Consequently, the reliability of frequent-occurrence time slots to be displayed can be increased.

The excluding unit f according to each of the embodiments is configured such that any item is selectable and specifiable to be excluded from the object of search. Therefore, the medical worker can precisely determine whether the exclusion of the items by the excluding unit f matches the purpose of extraction. Consequently, the reliability of information to be extracted and displayed can be increased further. In particular, if the excluding unit f is configured such that items to be excluded from the object of search are specified in units of one history, the entirety of the history of a single treatment session can be excluded from the object of search. Hence, information that is unfavorable for performing highly accurate extraction can be excluded in units of one treatment session. Furthermore, the excluding unit f is configured such that items to be excluded from the object of search are specified in units of one piece of data included in each of the histories. Therefore, the object of exclusion can be specified more precisely.

While some embodiments have been described above, the present invention is not limited thereto. For example, the blood purification apparatuses 1 may be connected to the server 3 of the managing apparatus 2 without using wires such as the LAN cables L (for example, the blood purification apparatuses 1 and the server 3 may be capable of wireless communication with each other). Moreover, the server 3 of the managing apparatus 2 that is connected to the blood purification apparatuses 1 may also be connected to any other independent server installed in a room other than the room related to dialysis treatment (for example, to a server included in any of various systems such as a system storing treatment information (electronic medical charts) and medication information of any other department in the same hospital (in a room different from the dialysis room, such as a room of an internal medicine concerning complications), a nursing support system provided for the nursing of patients, or a reservation or accounting system), so that the server 3 and the other server can transmit and receive various pieces of patient information to and from each other.

The display control unit 7 according to each of the above embodiments is capable of controlling information to be displayed on the display unit 4a of the client PC 4 during the current session of blood purification treatment. Alternatively, the display control unit 7 may control information to be displayed on the display unit 1a of a corresponding one of the blood purification apparatuses 1 during the current session of blood purification treatment. Furthermore, while each of the above embodiments concerns a case where the web-application module 3c for activating the client application 4b is provided in the server 3, the web-application module 3c may alternatively be provided in the client PC 4.

The above embodiments each concern a hemodialysis apparatus capable of performing a treatment such as hemodialysis (HD), ECUM, or HDF (hemodiafiltration). Alternatively, the present invention may be applied to a blood purification apparatus capable of performing another kind of blood purification treatment (such as hemofiltration (HF), continuous slow hemofiltration (CHF), or the like).

### Industrial Applicability

The present invention is applicable to any blood purification system, including those having other additional functions and so forth, as long as the blood purification system includes an extracting unit that searches for and extracts any history satisfying a predetermined condition from among histories stored in a storage unit, an excluding unit that excludes any of the histories stored in the storage unit from an object of search performed by the extracting unit, and a display control unit that controls various pieces of information based on the history extracted by the extracting unit to be displayed on a display unit.

### Reference Signs List

- 1: blood purification apparatus
- 1a: display unit
- 2: managing apparatus
- 3: server
- 3a: storage unit
- 3b: communication module
- 3c: web-application module
- 4: client PC
- 4a: display unit
- 5: extracting unit
- 6a: calculating unit
- 6b: estimating unit
- 6c: list-creating unit
- 7: display control unit
- 8: input unit
- 9: blood circuit
- 10: blood pump
- 11: dialyzer (blood purifier)
- 12: duplex pump
- 13: ultrafiltration pump
- 14: external indicator lamp
- R: frequent-occurrence time slot
- f: excluding unit

## Claims

1. A blood purification system comprising:
a blood purification apparatus that gives blood purification treatment to a patient;
a managing apparatus that communicates with the blood purification apparatus in such a manner as to transmit and receive information on the blood purification treatment to and from the blood purification apparatus, the managing apparatus including a storage unit that stores the information on the blood purification treatment as a history for each treatment session in a time course with progress of the treatment; and
a display unit provided to the blood purification apparatus or to the managing apparatus and that displays the information on the blood purification treatment,
**characterized in that** the blood purification system further includes
an extracting unit that searches for and extracts any history satisfying a predetermined condition from among the histories stored in the storage unit;
an excluding unit that excludes any of the histories stored in the storage unit from an object of search performed by the extracting unit; and
a display control unit that controls various pieces of information based on the history extracted by the extracting unit to be displayed on the display unit,
wherein the histories each include a particular incident related to the patient's behavior or the patient's condition having occurred unsteadily in the blood purification treatment, and
wherein the extracting unit is capable of extracting the particular incident by searching the histories stored in the storage unit except the history excluded by the excluding unit; the blood purification system includes a calculating unit that calculates a time when the particular incident extracted by the extracting unit occurs a predetermined or more number of times as a frequent-occurrence time slot; and the display control unit controls the frequent-occurrence time slot calculated by the calculating unit to be displayed on the display unit during a current session of blood purification treatment.

2. The blood purification system according to Claim 1, wherein the excluding unit is configured such that any item to be excluded from the object of search is specified.

3. The blood purification system according to Claim 2, wherein the excluding unit is configured such that the item to be excluded from the object of search is specified in units of one history.

4. The blood purification system according to Claim 2, wherein the excluding unit is configured such that the item to be excluded from the object of search is specified in units of one piece of data included in each of the histories.

## Patentansprüche

1. Ein Blutreinigungssystem umfassend:
einen Blutreinigungsapparat, der eine Blutreinigungsbehandlung an einem Patienten durchführt;
eine Verwaltungseinrichtung, die mit dem Blutreinigungsapparat in der Weise kommuniziert, dass sie Informationen über die Blutreinigungsbehandlung an den Blutreinigungsapparat sendet und
von diesem empfängt, wobei die Verwaltungseinrichtung einen Speicher einschließt, der die Informationen über die Blutreinigungsbehandlung als eine Historie für jede Behandlungssitzung in einem zeitlichen Verlauf mit dem Fortschritt der Behandlung speichert; und
eine Anzeigeeinheit, die an dem Blutreinigungsapparat oder an der Verwaltungsvorrichtung vorgesehen ist und die die Informationen über die Blutreinigungsbehandlung anzeigt,
**dadurch gekennzeichnet, dass** das Blutreinigungssystem weiterhin einschließt eine Extraktionseinheit, die unter den in dem Speicher gespeicherten Historien jede Historie sucht und extrahiert, die eine vorbestimmte Bedingung erfüllt;
eine Ausschlusseinheit, die irgendeine der in dem Speicher gespeicherten Historien von einem von der Extraktionseinheit durchgeführten Suchobjekt ausschließt; und
eine Anzeigesteuereinheit, die auf der Basis der von der Extraktionseinheit extrahierten Historie verschiedene Informationen steuert, um auf der Anzeigeeinheit angezeigt zu werden,
wobei die Historien jeweils ein bestimmtes Ereignis einschließen, das sich auf das Verhalten des Patienten oder den Zustand des Patienten bezieht, das bei der Blutreinigungsbehandlung unregelmäßig aufgetreten ist, und
wobei die Extraktionseinheit in der Lage ist, das bestimmte Ereignis zu extrahieren, indem sie die in der Speichereinheit gespeicherten Historien mit Ausnahme der von der Ausschlusseinheit ausgeschlossenen Historien durchsucht; das Blutreinigungssystem eine Berechnungseinheit einschließt, die eine Zeit, in der das von der Extraktionseinheit extrahierte bestimmte Ereignis eine vorbestimmte Anzahl von Malen oder häufiger auftritt, als ein Häufigkeitszeitfenster berechnet; und die Anzeigesteuereinheit das von der Berechnungseinheit berechnete Häufigkeitszeitfenster steuert, um während einer aktuellen Sitzung der Blutreinigungsbehandlung auf der Anzeigeeinheit angezeigt zu werden.

2. Blutreinigungssystem nach Anspruch 1, wobei die Ausschlusseinheit so ausgebildet ist, dass jeder aus dem Suchobjekt auszuschließende Gegenstand spezifiziert wird.

3. Blutreinigungssystem nach Anspruch 2, wobei die Ausschlusseinheit so ausgebildet ist, dass der vom Suchobjekt auszuschließende Gegenstand in Einheiten von einer Historie spezifiziert wird.

4. Blutreinigungssystem nach Anspruch 2, wobei die Ausschlusseinheit so ausgebildet ist, dass der vom Suchobjekt auszuschließende Gegenstand in Einheiten eines in jeder der Historien enthaltenen Datenstücks spezifiziert wird.

## Revendications

1. Système de purification de sang comprenant :
un appareil de purification de sang destiné à donner un traitement de purification de sang à un patient ;
un appareil de gestion destiné à communiquer avec ledit appareil de purification de sang de manière à transmettre et recevoir des informations sur le traitement de purification de sang vers et depuis ledit appareil de purification de sang, ledit appareil de gestion comprenant une unité de stockage destinée à stocker les informations sur le traitement de purification de sang en tant que historique pour chaque session de traitement dans un déroulement chronologique selon le progrès du traitement ; et
une unité d'affichage qui est prévue dans ledit appareil de purification ou dans ledit appareil de gestion et qui est destinée à afficher les informations sur le traitement de purification de sang,
**caractérisé en ce que** le système de purification de sang comprend en outre une unité d'extraction destinée à rechercher et extraire toute historique satisfaisante une condition prédéterminée parmi les historiques stockées dans ladite unité de stockage ;
une unité d'exclusion destinée à exclure une historique quelconque des historiques stockées dans ladite unité de stockage de l'objet de recherche effectué par ladite unité d'extraction ; et une unité de commande d'affichage destinée à contrôler des informations diverses sur la base de l'historique extraite par ladite unité d'extraction pour être affichées sur ladite unité d'affichage,
chacune des historiques comportant un évènement particulier concernant le comportement du patient ou la condition du patient qui s'est produit de façon irrégulière dans le traitement de purification de sang, et
ladite unité d'extraction étant apte à extraire l'évènement particulier en recherchant les historiques stockées dans ladite unité de stockage à l'exception de l'historique exclue par ladite unité d'exclusion ; ledit système de purification de sang comprenant une unité de calcul destinée à calculer une période où l'évènement particulier extrait par ladite unité d'extraction se produit pour un nombre prédéterminé ou plus de fois en tant qu'intervalle de temps d'événement fréquent, et ladite unité de commande d'affichage étant destinée à commander ladite intervalle de temps d'événement fréquent calculée par ladite unité de calcul à être affichée sur ladite unité d'affichage pendant une session actuelle de traitement de purification de sang.

2. Système de purification de sang selon la revendication 1, dans lequel ladite unité d'extraction est configurée de façon que toute élément qui est à exclure de l'objet de recherche est spécifié.

3. Système de purification de sang selon la revendication 2, dans lequel ladite unité d'extraction est configurée de façon que toute élément qui est à exclure de l'objet de recherche est spécifié dans des unités d'une historique.

4. Système de purification de sang selon la revendication 2, dans lequel ladite unité d'extraction est configurée de façon que toute élément qui est à exclure de l'objet de recherche est spécifié dans des unités d'une donnée comprise dans chacune des historiques.
